# EUROPÄISCHE PATENTSCHRIFT

(11) **EP 2 379 487 B1**
(45) Veröffentlichungstag und Bekanntmachung des Hinweises auf die Patenterteilung: **23.01.2013**
(21) Anmeldenummer: 09771717.7
(22) Anmeldetag: 04.12.2009
(51) Int. Cl.: C07C 209/72, C07C 211/36

(54) **VERFAHREN ZUR HERSTELLUNG VON BIS(PARA-AMINOCYCLOHEXYL)METHAN**
PROCESS FOR PREPARING BIS(PARA-AMINOCYCLOHEXYL)METHANE
PROCÉDÉ DE FABRICATION DE BIS(PARA-AMINOCYCLOHEXYL)MÉTHANE

(30) Priorität: 20.12.2008 DE 102008064280
(43) Veröffentlichungstag der Anmeldung: 26.10.2011
(73) Patentinhaber: Bayer Intellectual Property GmbH, 40789 Monheim (DE)
(72) Erfinder: HIZALER HOFFMANN, Evin, 50733 Köln (DE); MLECZKO, Leslaw, 41542 Dormagen (DE); SCHELLEN, Ralph, 41541 Dormagen (DE); SCHUBERT, Stephan, League City, 77573 Texas (US)
(74) Vertreter: BIP Patents
(86) Internationale Anmeldenummer: PCT/EP2009/008669
(87) Internationale Veröffentlichungsnummer: WO 2010/069484

(56) Entgegenhaltungen:
- EP-A1- 1 566 372
- WO-A1-01/54806
- ELVERS BARBARA ET AL: "Ullmanns Encyclopedia of Industrial Chemistry, Passages" ULLMANNS ENCYCLOPEDIA OF INDUSTRIAL CHEMISTRY, PRINCIPLES OF CHEMICAL REACTION ENGINEERING AND PLANT DESIGN, VCH, WEINHEIM ; BASEL ; CAMBRIDGE ; NEW YORK, Bd. B4, 1. Januar 1992 (1992-01-01), Seiten 95-104,210, XP002551481 ISBN: 978-3-527-20134-1 in der Anmeldung erwähnt

## Beschreibung

Die vorliegende Erfindung betrifft ein Verfahren zur Herstellung von Bis(Para-Aminocyclohexyl)Methan durch Mehrphasenreaktion von Methylendianilin mit Wasserstoff, worin die Umsetzung in 5 bis 50 hintereinander geschalteten Reaktionszonen unter adiabaten Bedingungen durchgeführt wird.

Bis(Para-Aminocyclohexyl)Methan (PACM) wird allgemein unter katalytischem Einfluss von Übergangsmetallkatalysatoren wie etwa Rutheniumkatalysatoren oder Rhodiumkatalysatoren aus einer flüssigen Phase umfassend Methylendianilin und einer gasförmigem Phase umfassend Wasserstoff in einer exothermen, katalytischen Reaktion gemäß Formel (I) hergestellt:

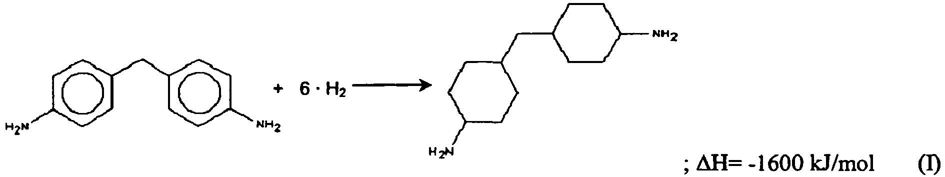

Das mittels der Reaktion nach Formel (I) hergestellte PACM ist ein Zwischenprodukt bei der Herstellung von Polyurethan, das wiederum eine große Bandbreite an Verwendungsmöglichkeiten besitzt.

So findet das resultierende Polyurethan etwa als Basis für Lacke Anwendung. Alternativ kann es aufgeschäumt werden und so als Polyurethanschaum, etwa als Dämmstoff oder als Polstermittel, verwendet werden.

Bei der Reaktion gemäß Formel (I) wird eine beträchtliche Menge an Wärme freigesetzt, die zunächst in der flüssigen Phase absorbiert wird und aus derselben wieder abgeführt werden muss, um etwa eine Verdampfung der flüssigen Phase zu verhindern. Weiter muss die Temperatur solcher Reaktionen kontrolliert werden, um die Möglichkeit explosionsartiger Nebenreaktionen, wie sie dem Fachmann im Zusammenhang mit der Aromatenchemie geläufig sind, zu verhindern.

In der US 6,998,507 B1 wird ein Verfahren zur Herstellung von PACM offenbart, das in einer Vorstufe auch die Herstellung des Methylendianilins umfasst. Die Herstellung von PACM gemäß dem Gegenstand dieser Erfindung wird in einem Reaktor ausgeführt, der einen monolithischen Katalysator in einem gerührten Behälter umfasst. Der Katalysator ist vorzugsweise ein Katalysator aus einem Rhodium/Ruthenium-Bimetall-Katalysator.

Die Offenbarung der US 6,998,507 B1 umfasst keinen Hinweis auf eine eventuelle adiabate Betriebsweise der Reaktionszone, wenngleich eine Vorkühlung des Methylendianilins vor Eintritt in die Reaktionszone zur Herstellung von PACM offenbart wird.

Das Verfahren ist nachteilig, weil im Falle des Versagens dieser nur einfach vorgesehenen Kühlung gemäß der US 6,998,507 B1 die Reaktion unkontrolliert durchgeht und die zuvor geschilderten explosionsgefährlichen Betriebszustände erreicht werden können. Eine verlässliche und genaue Temperaturkontrolle ist mit dem in der US 6,998,507 B1 offenbarten Verfahren weiter auch alleine daher schon nicht möglich, da beträchtliche Volumina der Reaktionszone nicht in unmittelbarem Kontakt mit einer Wärme austauschenden Fläche stehen, so dass die Bildung von nicht kontrollierten Temperaturgradienten innerhalb der Reaktionszone wahrscheinlich ist, was wahrscheinlich auch den Grund für die im Wesentlichen absatzweise Betriebsweise nach dem Verfahren der US 6,998,507 B1 bildet.

Ein weiteres Verfahren für allgemeine Hydrierungen, zu denen auch die hier betrachtete Hydrierung von Methylendianilin zu PACM gemäß der Formel (I) zählt, offenbart die EP 1 566 372 A1, wobei in dem hier offenbarten Verfahren Rieselbett-Anordnungen in Rohren offenbart werden. Die hierdurch entstehenden Reaktionszonen können mit verschiedenen Katalysatormaterialien befüllt sein. Die EP 1 566 372 offenbart weiter, dass nur vor und/oder nach einer Anzahl von drei in Reihe geschalteten Reaktionszonen eine Kühlung erfolgen kann.

Eine konkrete Offenbarung hinsichtlich einer adiabaten oder isothermen Betriebsweise enthält die EP 1 566 372 A1 nicht. Es ist in Ermangelung einer Offenbarung hinsichtlich Wärmeisolation um die Reaktionszonen und in Anbetracht des hohen Oberflächen- zu Volumenverhältnisses der Rohr-Anordnung gemäß der EP 1 566 372 A1 davon auszugehen, dass es gewünscht ist, Wärme über die Oberflächen der Rohre abzuführen, was zu einer eher polytropen Betriebsweise des Verfahrens führt.

Das Verfahren nach der Offenbarung der EP 1 566 372 A1 ist nachteilig, da ebenso, wie im Falle des Verfahrens nach der US 6,998,507 B1, unsichere Betriebszustände der stark exothermen Reaktion gemäß der Formel (I) nicht hinreichend kontrolliert werden können, da eine Kühlung der Reaktionspartner nur vor und/oder hinter einer Mehrzahl von Reaktionszonen vorgesehen wird. Außerdem wird die Reaktionswärme in Ermangelung von Maßnahmen zu deren Aufnahme in die Umgebung dissipiert und ist somit verloren, was unwirtschaftlich ist. Mit der mangelnden Temperaturkontrolle des Verfahrens nach der EP 1 566 372 A1 geht weiter einher, dass die gewünschten Selektivitäten und Ausbeuten an PACM nur schwer eingehalten werden können, wenn es zu Betriebsstörungen kommt.

Ein drittes Verfahren zur Herstellung von PACM offenbart die US 5,196,594 A, nach dem in mindestens einem Festbettreaktor bei Temperaturen von 100°C bis 190°C unter einem Druck von 50 bis 350 bar aus Methylendianilin mit Wasserstoff unter anderem PACM gebildet werden kann.

Die maximale Anzahl an gemäß der Offenbarung nach der US 5,196,594 A in Reihe verwendbaren Reaktionszonen beträgt zwei, wobei zwischen den Reaktionszonen keine Kühlung offenbart wird. Weiter wird keine konkrete Offenbarung zur Betriebsweise gegeben, nach der eine Unterscheidung zwischen einer bevorzugt isothermen oder adiabaten Verfahrensweise ableitbar wäre.

Das Verfahren nach der US 5,196,594 A weist aber mindestens die Nachteile der Verfahren nach den Offenbarungen der EP 1 566 372 A1 und US 6,998,507 B1 auf, da auch hier eine ausreichende Temperaturkontrolle nicht erzielt werden kann, was wiederum mindestens zu einem Gefährdungspotential oder zu unzureichenden Ausbeuten/Selektivitäten führt.

In EP 1 251 951 (B1) wird eine Vorrichtung und die Möglichkeit der Durchführung chemischer Reaktionen in der Vorrichtung offenbart, wobei die Vorrichtung durch eine Kaskade aus miteinander in Kontakt stehenden Reaktionszonen und Wärmetauschervorrichtungen gekennzeichnet ist, die stoffschlüssig miteinander im Verbund angeordnet sind. Das hierin durchzuführende Verfahren ist also gekennzeichnet durch den Kontakt der verschiedenen Reaktionszonen mit einer jeweiligen Wärmetauschervorrichtung in Form einer Kaskade.

Eine Offenbarung hinsichtlich der Verwendbarkeit der Vorrichtung und des Verfahrens zur Synthese von PACM aus flüssigem Methylendianilin und gasförmigem Wasserstoff findet nicht statt. Insbesondere wird eine Anwendbarkeit auf mehrphasige Reaktionssysteme im Allgemeinen nicht offenbart.

Es bleibt also unklar, wie, ausgehend von der Offenbarung der EP 1 251 951 (B1), eine solche Reaktion mittels der Vorrichtung und des darin ausgeführten Verfahrens durchgeführt werden soll. Weiter muss aus Gründen der Einheitlichkeit davon ausgegangen werden, dass das in der EP 1 251 951 (B1) offenbarte Verfahren in einer Vorrichtung gleich oder ähnlich der Offenbarung bezüglich der Vorrichtung ausgeführt wird. Hieraus resultiert, dass durch den offenbarungsgemäßen großflächigen Kontakt der Wärmeaustauschzonen mit den Reaktionszonen eine signifikante Menge an Wärme durch Wärmeleitung zwischen den Reaktionszonen und den benachbarten Wärmeaustauschzonen übertragen wird. Die Offenbarung hinsichtlich des oszillierenden Temperaturprofils kann also nur so verstanden werden, dass die hier festgestellten Temperaturspitzen stärker ausfallen würden, wenn dieser Kontakt nicht bestehen würde. Ein weiteres Indiz hierfür ist der exponentielle Anstieg der offenbarten Temperaturprofile zwischen den einzelnen Temperaturspitzen. Diese deuten an, dass eine gewisse Wärmesenke mit merklicher, aber begrenzter Kapazität in jeder Reaktionszone vorhanden ist, die den Temperaturanstieg in derselben reduzieren kann. Es kann nie ausgeschlossen werden, dass eine gewisse Abfuhr von Wärme (z.B. durch Strahlung) stattfindet; allerdings würde sich bei einer Reduktion der möglichen Wärmeabfuhr aus der Reaktionszone ein linearer oder in seiner Steigung degressiver Temperaturverlauf andeuten, da keine Nachdosierung von Edukten vorgesehen ist und somit nach exothermer Abreaktion die Reaktion immer langsamer und sich somit die erzeugte Wärmetönung verringern würde. Somit offenbart die EP 1 251 951 (B1) mehrstufige Verfahren in Kaskaden von Reaktionszonen, aus denen Wärme in undefinierter Menge durch Wärmeleitung abgeführt wird. Demnach ist das offenbarte Verfahren dahingehend nachteilig, als das eine genaue Temperaturkontrolle der Prozessgase der Reaktion nicht möglich ist.

Ausgehend vom Stand der Technik wäre es daher vorteilhaft, ein Verfahren bereitzustellen, das in einfachen Reaktionsvorrichtungen durchgeführt werden kann und das eine genaue, einfache Temperaturkontrolle ermöglicht, so dass es hohe Umsätze bei möglichst hohen Reinheiten des Produktes PACM erlaubt.

Für die Herstellung von PACM aus Methylendianilin mittels katalytischer Hydrierung wurden wie gerade dargestellt, bisher noch keine geeigneten Verfahren offenbart, die die zuvor genannten Aufgaben in ihrer Gesamtheit zu lösen vermögen.

Es besteht daher die Aufgabe, ein Verfahren zur katalytischen Hydrierung von Methylendianilin zu PACM bereitzustellen, das unter genauer Temperaturkontrolle in einfachen Reaktionsvorrichtungen durchführbar ist und das hierdurch hohe Umsätze bei hohen Reinheiten des Produktes erlaubt, wobei die Reaktionswärme entweder zu Gunsten der Reaktion oder in anderer Weise genutzt werden kann.

Es wurde überraschend gefunden, dass ein Verfahren zur Herstellung von Bis(Para-Aminocyclohexyl)Methan (PACM) aus Methylendianilin, befindlich in einer flüssigen Phase, und Wasserstoff, befindlich in einem Prozessgas, in Gegenwart von heterogenen Katalysatoren, dadurch gekennzeichnet, dass es in 5 bis 50 hintereinander geschalteten Reaktionszonen, in denen die heterogenen Katalysatoren vorliegen, unter adiabaten Bedingungen ausgeführt wird, diese Aufgabe zu lösen vermag.

Methylendianilin bezeichnet im Zusammenhang mit der vorliegenden Erfindung eine Flüssigkeit als Bestandteil einer flüssigen Phase, die in das erfindungsgemäße Verfahren eingeführt wird und die im Wesentlichen Methylendianilin umfasst. Üblicherweise liegt der Anteil an Methylendianilin an der dem Verfahren zugeführten flüssigen Phase zwischen 90 und 100 Gew.-%, bevorzugt zwischen 95 und 100 Gew.%.

Wasserstoff bezeichnet im Zusammenhang mit der vorliegenden Erfindung ein Prozessgas, das in das erfindungsgemäße Verfahren eingeführt wird und das im Wesentlichen Wasserstoff umfasst. Üblicherweise liegt der Anteil an Wasserstoff an den, dem Verfahren zugeführten Prozessgasen zwischen 90 und 100 Gew.%, bevorzugt zwischen 95 und 100 Gew.-%.

Neben der wesentlichen Komponente der flüssigen Phase Methylendianilin, kann diese auch noch weitere Nebenkomponenten umfassen. Nicht abschließende Beispiele für weitere Nebenkomponenten, die in der flüssigen Phase enthalten sein können, sind etwa Bis(Para-Aminocyclohexyl)-Methan, 4-(4'-Aminobenzyl)cyclohexylamin, trans,trans-Bis(Para-Aminocyclohexyl)Methan, sowie gelöste Bestandteile von Prozessgas.

Neben der wesentlichen Komponente des Prozessgases Wasserstoff, kann dieses auch noch Nebenkomponenten umfassen. Nicht abschließende Beispiele für Nebenkomponenten, die in dem Prozessgas enthalten sein können, sind etwa Argon, Stickstoff und/oder Kohlendioxid.

Allgemein wird im Zusammenhang mit der vorliegenden Erfindung ein Prozessgas als ein Gasgemisch verstanden, das im Wesentlichen Wasserstoff und Nebenkomponenten umfasst, während eine flüssige Phase als Flüssigkeit und/oder Gemisch von Flüssigkeiten und/oder als eine Lösung verstanden wird, die Methylendianilin und weitere Nebenkomponenten umfasst.

Im Wesentlichen bedeutet also gemäß der vorstehenden Definition, dass ein Anteil der im Wesentlichen enthaltenen Komponente an der flüssigen Phase und/oder an dem Prozessgas von mehr als 90 Gew.% vorliegt.

Erfindungsgemäß bedeutet die Durchführung des Verfahrens unter adiabaten Bedingungen, dass der Reaktionszone von außen im Wesentlichen weder aktiv Wärme zugeführt noch Wärme entzogen wird. Es ist allgemein bekannt, dass eine vollständige Isolation gegen Wärmezu- oder Abfuhr nur durch vollständige Evakuierung unter Ausschluss der Möglichkeit des Wärmeübergangs durch Strahlung möglich ist. Daher bezeichnet adiabat im Zusammenhang mit der vorliegenden Erfindung, dass keine Maßnahmen zur Wärmezu- oder -abfuhr ergriffen werden.

In einer alternativen Ausführungsform des erfindungsgemäßen Verfahrens kann aber z.B. durch Isolation mittels allgemein bekannter Isolationsmittel, wie z.B. Polystyroldämmstoffen, oder auch durch genügend große Abstände zu Wärmesenken oder Wärmequellen, wobei das Isolationsmittel Luft ist, ein Wärmeübergang vermindert werden.

Ein Vorteil der erfindungsgemäßen adiabaten Fahrweise der 5 bis 50 hintereinander geschalteten Reaktionszonen gegenüber einer nicht adiabaten Fahrweise besteht darin, dass in den Reaktionszonen keine Mittel zur Wärmeabfuhr bereitgestellt werden müssen, was eine erhebliche Vereinfachung der Konstruktion mit sich bringt. Dadurch ergeben sich insbesondere Vereinfachungen bei der Fertigung des Reaktors sowie bei der Skalierbarkeit des Verfahrens und eine Steigerung der Reaktionsumsätze. Außerdem kann die Wärme, die im Zuge des exothermen Reaktionsfortschrittes erzeugt wird, in der einzelnen Reaktionszone zur Steigerung des Umsatzes in kontrollierter Weise genutzt werden.

Ein weiterer Vorteil des erfindungsgemäßen Verfahrens ist die Möglichkeit der sehr genauen Temperaturkontrolle, durch die enge Staffelung von adiabaten Reaktionszonen. Es kann somit in jeder Reaktionszone eine im Reaktionsfortschritt vorteilhafte Temperatur eingestellt und kontrolliert werden.

Die im erfindungsgemäßen Verfahren verwendeten Katalysatoren sind üblicherweise Katalysatoren, die aus einem Material bestehen, das neben seiner katalytischen Aktivität für die Reaktion gemäß Formel (I) durch ausreichende chemische Resistenz unter den Bedingungen des Verfahrens, sowie durch eine hohe spezifische Oberfläche gekennzeichnet sind.

Katalysatormaterialien, die durch eine solche chemische Resistenz unter den Bedingungen des Verfahrens gekennzeichnet sind, sind zum Beispiel Katalysatoren umfassend Ruthenium und/oder Rhenium und/oder Rhodium. Zumeist sind diese Materialien auf Träger, die Oxide von Aluminium und/oder Titan und/oder Silizium umfassen, aufgebracht.

Spezifische Oberfläche bezeichnet im Zusammenhang mit der vorliegenden Erfindung die Fläche des Katalysatormaterials, die vom Prozessgas erreicht werden kann, bezogen auf die eingesetzte Masse an Katalysatormaterial.

Eine hohe spezifische Oberfläche ist eine spezifische Oberfläche von mindestens 10 m²/g, bevorzugt von mindestens 20 m²/g.

Die erfindungsgemäßen Katalysatoren befmden sich jeweils in den Reaktionszonen und können in allen an sich bekannten Erscheinungsformen, z.B. Festbett und Wanderbett, vorliegen.

Die Erscheinungsform als Festbett ist für das erfindungsgemäße Verfahren bevorzugt.

Die Festbettanordnung umfasst eine Katalysatorschüttung im eigentlichen Sinn, d. h. losen, geträgerten oder ungeträgerten Katalysator in beliebiger Form sowie in Form von geeigneten Packungen. Der Begriff der Katalysatorschüttung, wie er hier verwendet wird, umfasst auch zusammenhängende Bereiche geeigneter Packungen auf einem Trägermaterial oder strukturierte Katalysatorträger. Dies wären z.B. zu beschichtende keramische Wabenträger mit vergleichsweise hohen geometrischen Oberflächen oder gewellte Schichten aus Metalldrahtgewebe, auf denen beispielsweise Katalysatorgranulat immobilisiert ist. Als eine Sonderform der Packung wird im Zusammenhang mit der vorliegenden Erfindung das Vorliegen des Katalysators in monolithischer Form betrachtet.

Wird eine Festbettanordnung des Katalysators verwendet, so liegt der Katalysator bevorzugt in Schüttungen von Partikeln mit mittleren Partikelgrößen von 1 bis 10 mm, bevorzugt 1,5 bis 8 mm, besonders bevorzugt von 2 bis 6 mm vor.

Wird eine Wanderbettanordnung des Katalysators verwendet, so liegt der Katalysator bevorzugt in losen Schüttungen von Partikeln vor, wie sie im Zusammenhang mit der Festbettanordnung bereits beschrieben worden sind.

Schüttungen von solchen Partikeln sind vorteilhaft, weil die Partikel eine hohe spezifische Oberfläche des Katalysatormaterials gegenüber der flüssigen Phase, als auch dem Prozessgas besitzen und damit eine hohe Umsatzrate erreicht werden kann. Es kann also die Stofftransportlimitierung der Reaktion durch Diffusion gering gehalten werden. Zugleich sind die Partikel damit aber noch nicht so klein, dass es zu überproportional erhöhten Druckverlusten bei Durchströmung des Festbettes kommt. Die Bereiche der in der bevorzugten Ausführungsform des Verfahrens, umfassend eine Reaktion in einem Festbett, angegebenen Partikelgrößen sind somit ein Optimum zwischen dem erreichbaren Umsatz aus der Reaktion gemäß Formel (I) und dem erzeugten Druckverlust bei Durchführung des Verfahrens. Druckverlust ist in direkter Weise mit der notwendigen Energie in Form von Pumpen- und/oder Kompressorleistung gekoppelt, so dass eine überproportionale Erhöhung desselben in einer unwirtschaftlichen Betriebsweise des Verfahrens resultieren würde.

In einer bevorzugten Ausfühmngsform des erfindungsgemäßen Verfahrens erfolgt der Umsatz in 10 bis 40, besonders bevorzugt 15 bis 35 hintereinander geschalteten Reaktionszonen.

Eine bevorzugte weitere Ausführungsform des Verfahrens ist dadurch gekennzeichnet, dass das aus mindestens einer Reaktionszone austretende Prozessgas und die flüssige Phase anschließend durch wenigstens eine dieser Reaktionszone nachgeschalteten Wärmeaustauschzone geleitet wird.

In einer besonders bevorzugten weiteren Ausführungsform des Verfahrens befindet sich nach jeder Reaktionszone wenigstens eine, bevorzugt genau eine Wärmeaustauschzone, durch die das aus der Reaktionszone austretende Prozessgas und die flüssige Phase geleitet wird.

Die Reaktionszonen können dabei entweder in einem Reaktor angeordnet oder in mehreren Reaktoren aufgeteilt angeordnet werden. Die Anordnung der Reaktionszonen in einem Reaktor führt zu einer Verringerung der Anzahl der verwendeten Apparaturen.

Die einzelnen Reaktionszonen und Wärmeaustauschzonen können auch zusammen in einem Reaktor oder in beliebigen Kombinationen von jeweils Reaktionszonen mit Wärmeaustauschzonen in mehreren Reaktoren aufgeteilt, angeordnet werden.

Liegen Reaktionszonen und Wärmeaustauschzonen in einem Reaktor vor, so befindet sich in einer alternativen Ausführungsform der Erfindung zwischen diesen eine Wärmeisolationszone, um den adiabaten Betrieb der Reaktionszone erhalten zu können.

Zusätzlich können einzelne der in Reihe geschalteten Reaktionszonen unabhängig voneinander auch durch eine oder mehrere parallel geschaltete Reaktionszonen ersetzt oder ergänzt werden. Die Verwendung von parallel geschalteten Reaktionszonen erlaubt insbesondere deren Austausch bzw. Ergänzung bei laufendem kontinuierlichen Gesamtbetrieb des Verfahrens.

Parallele und hintereinander geschaltete Reaktionszonen können insbesondere auch miteinander kombiniert sein. Besonders bevorzugt weist das erfindungsgemäße Verfahren aber ausschließlich hintereinander geschaltete Reaktionszonen auf.

Die im erfindungsgemäßen Verfahren bevorzugt verwendeten Reaktoren können aus einfachen Behältern mit einer oder mehreren Reaktionszonen bestehen, wie sie z.B. in Ullmanns Encyclopedia of Industrial Chemistry (Fifth, Completely Revised Edition, Vol B4, Seite 95-104, Seite 210-216) beschrieben werden, wobei jeweils zwischen den einzelnen Reaktionszonen und/oder Wärmeaustauschzonen Wärmeisolationszonen zusätzlich vorgesehen sein können.

In einer alternativen Ausführungsform des Verfahrens, befindet sich also zwischen einer Reaktionszone und einer Wärmeaustauschzone mindestens eine Wärmeisolationszone. Bevorzugt befindet sich um jede Reaktionszone eine Wärmeisolationszone.

Die Katalysatoren bzw. die Festbetten daraus werden in an sich bekannter Weise auf oder zwischen gas- und flüssigkeitsdurchlässigen Wandungen umfassend die Reaktionszone des Reaktors angebracht. Insbesondere bei dünnen Festbetten können in Strömungsrichtung vor den Katalysatorbetten technische Vorrichtungen zur gleichmäßigen Gas- und/oder Flüssigkeitsverteilung angebracht werden. Dies können Lochplatten, Glockenböden, Ventilböden oder andere Einbauten sein, die durch Erzeugung eines geringen aber gleichmäßigen Druckverlusts einen gleichförmigen Eintritt des Prozessgases und/oder der flüssigen Phase in das Festbett bewirken.

In einer bevorzugten Ausführungsform des Verfahrens beträgt die Eingangstemperatur der in die erste Reaktionszone eintretenden flüssigen Phase von 10 bis 170°C, bevorzugt von 50 bis 150°C, besonders bevorzugt von 90 bis 140°C.

Wesentlich ist die Temperatur der flüssigen Phase, da diese, gemessen an dem Prozessgas eine um Größenordnungen höhere Wärmekapazität aufweist, so dass diese das gegebenenfalls wärmere oder kältere Prozessgas in der Reaktionszone in kurzer Zeit auf annähernd die gleiche Temperatur einstellt. Besonders bevorzugt hat aber das Prozessgas bei Eintritt in die jeweilige Reaktionszone ebenfalls die vorgenannte Temperatur.

In einer weiteren bevorzugten Ausführungsform des Verfahrens beträgt der absolute Druck am Eingang der ersten Reaktionszone mehr als 5 bar bevorzugt zwischen 10 und 200 ba, besonders bevorzugt zwischen 20 und 160 bar.

In noch einer weiteren bevorzugten Ausführungsform des Verfahrens beträgt die Verweilzeit des Prozessgases und der flüssigen Phase in allen Reaktionszonen zusammen zwischen 1 und 100 s, bevorzugt zwischen 2 und 80 s, besonders bevorzugt zwischen 5 und 50 s.

Die flüssige Phase und das Prozessgas werden bevorzugt nur vor der ersten Reaktionszone zugeführt. Dies hat den Vorteil, dass die gesamte flüssige Phase für die Aufnahme und Abfuhr der Reaktionswärme in allen Reaktionszonen genutzt werden kann. Außerdem kann durch eine solche Verfahrensweise die Raum-Zeit-Ausbeute gesteigert werden, bzw. die notwendige Katalysatormasse verringert werden. Es ist aber auch möglich vor einer oder mehreren der nach der ersten Reaktionszone folgenden Reaktionszonen nach Bedarf flüssige Phase umfassend Methylendianilin, bevorzugt reines flüssiges Methylendianilin, einzudosieren. Über die Zufuhr von flüssiger Phase zwischen den Reaktionszonen kann zusätzlich die Temperatur des Umsatzes gesteuert werden.

In einer bevorzugten Ausführungsform des erfindungsgemäßen Verfahrens werden die flüssige Phase und das Prozessgas nach mindestens einer der verwendeten Reaktionszonen, besonders bevorzugt nach jeder Reaktionszone, abgekühlt.

Dazu leitet man die flüssige Phase und das Prozessgas nach Austritt aus einer Reaktionszone durch eine oder mehrere der oben genannten Wärmeaustauschzonen, die sich hinter den jeweiligen Reaktionszonen befinden. Diese können als Wärmeaustauschzonen in Form der dem Fachmann bekannten Wärmetauscher, wie z.B. Rohrbündel-, Platten-, Ringnut-, Spiral-, Rippenrohr- und/oder mikrostrukturierte Wärmetauscher ausgeführt sein. Bevorzugt sind die Wärmetauscher mikrostrukturierte Wärmetauscher.

Mikrostrukturiert bezeichnet im Zusammenhang mit der vorliegenden Erfindung, dass der Wärmetauscher zum Zweck der Wärmeübertragung Fluid-führende Kanäle umfasst, die dadurch gekennzeichnet sind, dass sie einen hydraulischen Durchmesser zwischen 50 µm und 5 mm aufweisen. Der hydraulische Durchmesser berechnet sich aus dem Vierfachen der durchströmten Querschnittsfläche des Fluid-führenden Kanals dividiert durch den Umfang des Kanals.

In einer besonderen Ausführungsform des Verfahrens wird beim Abkühlen der flüssigen Phase und des Prozessgases in den Wärmeaustauschzonen durch den Wärmetauscher Dampf erzeugt.

Innerhalb dieser besonderen Ausführungsform ist es bevorzugt in den Wärmetauschern, die die Wärmeaustauschzonen beinhalten, auf der Seite des Kühlmediums eine Verdampfung, bevorzugt Teilverdampfung auszuführen.

Teilverdampfung bezeichnet im Zusammenhang mit der vorliegenden Erfindung eine Verdampfung, bei der ein Gas-/Flüssigkeitsgemisch eines Stoffes als Kühlmedium verwendet wird und bei der auch nach Wärmeübergang in dem Wärmetauscher noch ein Gas-/Flüssigkeitsgemisch eines Stoffes vorliegt.

Das Ausführen einer Verdampfung ist besonders vorteilhaft, weil hierdurch die erzielbaren Wärmedurchgangskoeffizienten von/zu Prozessgasen und flüssiger Phase auf/von Kühl-/Heizmedien besonders hoch werden und somit eine effiziente Kühlung erreicht werden kann.

Das Ausführen einer Teilverdampfung ist besonders vorteilhaft, weil die Aufnahme / Abgabe von Wärme durch das Kühlmedium hierdurch nicht mehr in einer Temperaturänderung des Kühlmediums resultiert, sondern lediglich das Gas-/Flüssig-Gleichgewicht verschoben wird. Das hat zur Folge, dass über die gesamte Wärmeaustauschzone die flüssige Phase und das Prozessgas gegenüber einer konstanten Temperatur gekühlt werden. Dies wiederum verhindert sicher das Auftreten von radialen Temperaturprofilen in der Strömung der flüssigen Phase und/oder Prozessgase, wodurch die Kontrolle über die Reaktionstemperaturen in den Reaktionszonen verbessert wird und insbesondere das Ausbilden von lokalen Überhitzungen durch radiale Temperaturprofile verhindert wird.

In einer alternativen Ausführungsform kann anstelle einer Verdampfung/Teilverdampfung auch eine Mischzone vor dem Eingang einer Reaktionszone vorgesehen werden, um die gegebenenfalls bei der Abkühlung entstehenden radialen Temperaturprofile in der Strömung der flüssigen Phase und/oder Prozessgase durch Vermischung quer zur hauptsächlichen Strömungsrichtung zu vereinheitlichen.

In einer bevorzugten Ausführungsform des Verfahrens werden die nacheinander geschalteten Reaktionszonen bei von Reaktionszone zu Reaktionszonen steigender oder sinkender Durchschnittstemperatur betrieben. Dies bedeutet, dass man innerhalb einer Folge von Reaktionszonen die Temperatur von Reaktionszone zu Reaktionszone sowohl ansteigen als auch absinken lassen kann. Dies kann beispielsweise über die Steuerung der zwischen die Reaktionszone geschalteten Wärmeaustauschzonen eingestellt werden. Weitere Möglichkeiten der Einstellung der Durchschnittstemperatur werden im Folgenden beschrieben.

Die Dicke der durchströmten Reaktionszonen kann gleich oder verschieden gewählt werden und ergibt sich nach dem Fachmann allgemein bekannten Gesetzmäßigkeiten aus der oben beschriebenen Verweilzeit und den jeweils im Verfahren durchgesetzten Mengen an flüssiger Phase und Prozessgas.

Die erfindungsgemäß mit dem Verfahren durchsetzbaren Massenströme an flüssiger Phase umfassend das Verfahrensprodukt (PACM), aus denen sich auch die einzusetzenden Mengen an Methylendianilin ergeben, liegen üblicherweise zwischen 0,01 und 30 t/h, bevorzugt zwischen 0,1 und 20 t/h, besonders bevorzugt zwischen 1 und 15 t/h.

Die maximale Austrittstemperatur der flüssigen Phase und/oder Prozessgase aus den Reaktionszonen liegt üblicherweise in einem Bereich von 130°C bis 250°C, bevorzugt von 140°C bis 230°C, besonders bevorzugt von 150°C bis 210°C. Die Steuerung der Temperatur in den Reaktionszonen erfolgt bevorzugt durch mindestens eine der folgenden Maßnahmen: Dimensionierung der adiabaten Reaktionszone, Steuerung der Wärmeabfuhr zwischen den Reaktionszonen, Zusatz von flüssiger Phase zwischen den Reaktionszonen, molares Verhältnis der Edukte / Überschuss an verwendetem Wasserstoff, Zusatz von Inertgasen, insbesondere Stickstoff, Kohlendioxid, vor und/oder zwischen den Reaktionszonen.

Die Zusammensetzung der Katalysatoren in den erfindungsgemäßen Reaktionszonen kann gleich oder verschieden sein. In einer bevorzugten Ausführungsform werden in jeder Reaktionszone die gleichen Katalysatoren verwendet. Man kann aber auch vorteilhaft verschiedene Katalysatoren in den einzelnen Reaktionszonen verwenden. So kann insbesondere in der ersten Reaktionszone, wenn die Konzentration der Reaktionsedukte noch hoch ist, ein weniger aktiver Katalysator verwendet werden und in den weiteren Reaktionszonen die Aktivität des Katalysators von Reaktionszone zu Reaktionszone gesteigert werden. Die Steuerung der Katalysatoraktivität kann auch durch Verdünnung mit Inertmaterialien bzw. Trägermaterial erfolgen. Ebenfalls vorteilhaft ist die Verwendung eines Katalysators insbesondere in der ersten und/oder zweiten Reaktionszone, der besonders stabil gegen eine Desaktivierung bei den Temperaturen des Verfahrens in diesen Reaktionszonen ist.

Mit dem erfindungsgemäßen Verfahren können pro 1 kg Katalysator 0,1 kg/h bis 50 kg/h, bevorzugt 1 kg/h bis 20 kg/h, besonders bevorzugt 2 kg/h bis 10 kg/h PACM hergestellt werden.

Das erfindungsgemäße Verfahren zeichnet sich somit durch hohe Raum-Zeit-Ausbeuten aus, verbunden mit einer Verringerung der Apparategrößen sowie einer Vereinfachung der Apparaturen bzw. Reaktoren. Diese überraschend hohe Raum-Zeit-Ausbeute wird durch das Zusammenspiel der erfindungsgemäßen und bevorzugten Ausführungsformen des neuen Verfahrens ermöglicht. Insbesondere das Zusammenspiel von gestaffelten, adiabaten Reaktionszonen mit dazwischen befindlichen Wärmeaustauschzonen und den definierten Verweilzeiten ermöglicht eine genaue Steuerung des Verfahrens und die daraus resultierenden hohen Raum-Zeit-Ausbeuten, sowie eine Verringerung des Risikos an explosionsgefährlichen Betriebszuständen und eine Vereinfachung des Verfahrens, da keine Katalysatorabtrennung und -rückführung erforderlich sind.

## Patentansprüche

1. Verfahren zur Herstellung von Bis(Para-Aminocyclohexyl)Methan (PACM) aus Methylendianilin, befindlich in einer flüssigen Phase, und Wasserstoff, befindlich in einem Prozessgas, in Gegenwart von heterogenen Katalysatoren, **dadurch gekennzeichnet, dass** es in 5 bis 50 hintereinander geschalteten Reaktionszonen, in denen die heterogenen Katalysatoren vorliegen, unter adiabaten Bedingungen ausgeführt wird.

2. Verfahren nach Anspruch 1, **dadurch gekennzeichnet, dass** der Umsatz in 10 bis 40, bevorzugt 15 bis 35 hintereinander geschalteten Reaktionszonen geschieht.

3. Verfahren nach Anspruch 1 oder Anspruch 2, **dadurch gekennzeichnet, dass** die Eingangstemperatur der in die erste Reaktionszone eintretenden flüssigen Phase von 10 bis 170°C, bevorzugt von 50 bis 150°C, besonders bevorzugt von 90 bis 140°C beträgt.

4. Verfahren nach Anspruch 1 bis 3, **dadurch gekennzeichnet, dass** der absolute Druck am Eingang der ersten Reaktionszone mehr als 5 bar, bevorzugt zwischen 10 und 200 bar, besonders bevorzugt zwischen 20 und 160 bar beträgt.

5. Verfahren nach einem der vorigen Ansprüche, **dadurch gekennzeichnet, dass** die Verweilzeit des Prozessgases und der flüssigen Phase in allen Reaktionszonen zusammen zwischen 1 und 100 s, bevorzugt zwischen 2 und 80 s, besonders bevorzugt zwischen 5 und 50 s beträgt.

6. Verfahren nach einem der vorigen Ansprüche, **dadurch gekennzeichnet, dass** die Katalysatoren in Festbettanordnung vorliegen.

7. Verfahren nach den Ansprüchen 1 bis 6, **dadurch gekennzeichnet, dass** die Katalysatoren in Schüttungen von Partikeln mit mittleren Partikelgrößen von 1 bis 10 mm, bevorzugt 1,5 bis 8 mm, besonders bevorzugt von 2 bis 6 mm vorliegen.

8. Verfahren nach einem der vorigen Ansprüche, **dadurch gekennzeichnet, dass** sich nach 25 mindestens einer Reaktionszone wenigstens eine Wärmeaustauschzone befindet, durch die das Prozessgas und die flüssige Phase geleitet werden.

9. Verfahren nach Anspruch 8, **dadurch gekennzeichnet, dass** sich nach jeder Reaktionszone wenigstens eine, bevorzugt eine Wärmeaustauschzone befindet, durch die das Prozessgas und die flüssige Phase geleitet werden.

10. Verfahren nach einem der vorigen Ansprüche, **dadurch gekennzeichnet, dass** sich zwischen einer Reaktionszone und einer Wärmeaustauschzone mindestens eine Wärmeisolationszone befindet.

11. Verfahren nach Anspruch 10, **dadurch gekennzeichnet, dass** sich um jede Reaktionszone eine Wärmeisolationszone befindet.

12. Verfahren nach einem der vorigen Ansprüche, **dadurch gekennzeichnet, dass** die Wärmeaustauschzonen als mikrostrukturierte Wärmetauscher ausgestaltet sind, in denen die Fluid-führende Kanäle einen hydraulischen Durchmesser zwischen 50 µm und 5 mm aufweisen.

13. Verfahren nach Anspruch 12, **dadurch gekennzeichnet, dass** beim Abkühlen der flüssigen Phase und des Prozessgases in den Wärmeaustauschzonen durch den Wärmetauscher auf der Seite des Kühlmediums Dampf erzeugt wird.

14. Verfahren nach einem der Ansprüche 12 oder 13, **dadurch gekennzeichnet, dass** in den Wärmetauschern auf der Seite des Kühlmediums eine Teilverdampfung ausgeführt wird.

15. Verfahren nach einem der vorigen Ansprüche, **dadurch gekennzeichnet, dass** die maximale Austrittstemperatur der flüssigen Phase und/oder Prozessgase aus den Reaktionszonen in einem Bereich von 130°C bis 250°C, bevorzugt von 140°C bis 230°C, besonders bevorzugt von 150°C bis 210°C liegt.

## Claims

1. Process for preparing bis(para-aminocyclohexyl)methane (PACM) from methylenedianiline present in a liquid phase and hydrogen present in a process gas, in the presence of heterogeneous catalysts, **characterized in that** it is performed in 5 to 50 series-connected reaction zones in which the heterogeneous catalysts are present under adiabatic conditions.

2. Process according to Claim 1, **characterized in that** the conversion is accomplished in 10 to 40 and preferably 15 to 35 series-connected reaction zones.

3. Process according to Claim 1 or Claim 2, **characterized in that** the inlet temperature of the liquid phase entering the first reaction zone is from 10 to 170°C, preferably from 50 to 150°C, more preferably from 90 to 140°C.

4. Process according to any of Claims 1 to 3, **characterized in that** the absolute pressure at the inlet of the first reaction zone is more than 5 bar, preferably between 10 and 200 bar, more preferably between 20 and 160 bar.

5. Process according to any of the preceding claims, **characterized in that** the residence time of the process gas and of the liquid phase in all reaction zones together is between 1 and 100 s, preferably between 2 and 80 s, more preferably between 5 and 50 s.

6. Process according to any of the preceding claims, **characterized in that** the catalysts are present in fixed bed arrangement.

7. Process according to any of Claims 1 to 6, **characterized in that** the catalysts are present in beds of particles with mean particle sizes of 1 to 10 mm, preferably 1.5 to 8 mm, more preferably of 2 to 6 mm.

8. Process according to any of the preceding claims, **characterized in that** downstream of at least one reaction zone is at least one heat exchange zone through which the process gas and the liquid phase are passed.

9. Process according to Claim 8, **characterized in that** downstream of each reaction zone is at least one, preferably one, heat exchange zone through which the process gas and liquid phase are passed.

10. Process according to any of the preceding claims, **characterized in that** between a reaction zone and a heat exchange zone is at least one thermal insulation zone.

11. Process according to Claim 10, **characterized in that** around each reaction zone is a thermal insulation zone.

12. Process according to any of the preceding claims, **characterized in that** the heat exchange zones are configured as microstructured heat exchangers in which the fluid-conducting channels have a hydraulic diameter between 50 µm and 5 mm.

13. Process according to Claim 12, **characterized in that** steam is raised on the side of the cooling medium as the liquid phase and the process gas are cooled in the heat exchange zones by the heat exchanger.

14. Process according to either of Claims 12 and 13, **characterized in that** a partial evaporation is performed in the heat exchangers, on the side of the cooling medium.

15. Process according to any of the preceding claims, **characterized in that** the maximum exit temperature of the liquid phase and/or process gases from the reaction zones is within a range from 130°C to 250°C, preferably from 140°C to 230°C, more preferably from 150°C to 210°C

## Revendications

1. Procédé de fabrication de bis(para-aminocyclohexyl)méthane (PACM) à partir de méthylènedianiline, se trouvant dans une phase liquide, et d'hydrogène, se trouvant dans un gaz de procédé, en présence de catalyseurs hétérogènes, **caractérisé en ce qu'**il est réalisé en conditions adiabatiques dans 5 à 50 zones de réaction connectées en série, dans lesquelles se trouvent les catalyseurs hétérogènes.

2. Procédé selon la revendication 1, **caractérisé en ce que** la transformation a lieu dans 10 à 40, de préférence 15 à 35, zones de réaction connectées en série.

3. Procédé selon la revendication 1 ou la revendication 2, **caractérisé en ce que** la température d'entrée de la phase liquide entrant dans la première zone de réaction est de 10 à 170 °C, de préférence de 50 à 150 °C, de manière particulièrement préférée de 90 à 140 °C.

4. Procédé selon les revendications 1 à 3, **caractérisé en ce que** la pression absolue à l'entrée de la première zone de réaction est supérieure à 5 bar, de préférence comprise entre 10 et 200 bar, de manière particulièrement préférée comprise entre 20 et 160 bar.

5. Procédé selon l'une quelconque des revendications précédentes, **caractérisé en ce que** le temps de séjour du gaz de procédé et de la phase liquide dans toutes les zones de réaction est au total compris entre 1 et 100 s, de préférence entre 2 et 80 s, de manière particulièrement préférée entre 5 et 50 s.

6. Procédé selon l'une quelconque des revendications précédentes, **caractérisé en ce que** les catalyseurs se présentent en un agencement à lit fixe.

7. Procédé selon les revendications 1 à 6, **caractérisé en ce que** les catalyseurs se présentent sous la forme de garnissages de particules de tailles de particules moyennes de 1 à 10 mm, de préférence de 1,5 à 8 mm, de manière particulièrement préférée de 2 à 6 mm.

8. Procédé selon l'une quelconque des revendications précédentes, **caractérisé en ce qu'**au moins une zone d'échange de chaleur, dans laquelle le gaz de procédé et la phase liquide sont conduits, se trouve après au moins une zone de réaction.

9. Procédé selon la revendication 8, **caractérisé en ce qu'**au moins une, de préférence une, zone d'échange de chaleur, dans laquelle le gaz de procédé et la phase liquide sont conduits, se trouve après chaque zone de réaction.

10. Procédé selon l'une quelconque des revendications précédentes, **caractérisé en ce qu'**au moins une zone d'isolation de la chaleur se trouve entre une zone de réaction et une zone d'échange de chaleur.

11. Procédé selon la revendication 10, **caractérisé en ce qu'**une zone d'isolation de la chaleur se trouve autour de chaque zone de réaction.

12. Procédé selon l'une quelconque des revendications précédentes, **caractérisé en ce que** les zones d'échange de chaleur se présentent sous la forme d'échangeurs de chaleur microstructurés, dans lesquels les canaux conduisant le fluide présentent un diamètre hydraulique compris entre 50 µm et 5 mm.

13. Procédé selon la revendication 12, **caractérisé en ce que** de la vapeur est générée par l'échangeur de chaleur du côté du milieu de refroidissement lors du refroidissement de la phase liquide et du gaz de procédé dans les zones d'échange de chaleur.

14. Procédé selon l'une quelconque des revendications 12 ou 13, **caractérisé en ce qu'**une évaporation partielle est réalisée dans les échangeurs de chaleur du côté du milieu de refroidissement.

15. Procédé selon l'une quelconque des revendications précédentes, **caractérisé en ce que** la température de sortie maximale de la phase liquide et/ou du gaz de procédé des zones de réaction se situe dans une plage allant de 130 °C à 250 °C, de préférence de 140 °C à 230 °C, de manière particulièrement préférée de 150 °C à 210 °C.
